# FASCICULE DE BREVET EUROPEEN

(11) **EP 4 416 160 B1**
(45) Date de publication et mention de la délivrance du brevet: **11.03.2026**
(21) Numéro de dépôt: 22802576.3
(22) Date de dépôt: 12.10.2022
(51) Int. Cl.: C07H 1/00, C07H 15/04, C07H 15/10

(54) **PROCÉDÉ DE PRÉPARATION D'ALKYLPOLYGLYCOSIDES FAIBLEMENT COLORÉS AVEC NEUTRALISATION DU MILIEU REACTIONNEL AVANT ÉLIMINATION DU SUCRE**
VERFAHREN ZUR HERSTELLUNG VON NIEDRIG GEFÄRBTEN ALKYLPOLYGLYKOSIDEN MIT NEUTRALISIERUNG DES REAKTIONSMEDIUMS VOR DER ENTFERNUNG DES ZUCKERS
METHOD FOR THE PREPARATION OF LOW COLORED ALKYLPOLYGLYCOSIDES WITH NEUTRALIZATION OF THE REACTION MEDIUM BEFORE REMOVAL OF THE SUGAR

(30) Priorité: 13.10.2021 FR 2110842
(43) Date de publication de la demande: 21.08.2024
(73) Titulaire: Société d'Exploitation de Produits pour les Industries Chimiques SEPPIC, 75321 Paris cedex 07 (FR)
(72) Inventeur: ILLOUS, Estelle, 81100 Castres (FR); DESSILLA, Stéphane, 81100 Castres (FR)
(74) Mandataire: Air Liquide
(86) Numéro de dépôt international: PCT/EP2022/078397
(87) Numéro de publication internationale: WO 2023/062074

(56) Documents cités:
- EP-A1- 0 077 167
- EP-A1- 0 092 876
- EP-A1- 0 338 151
- WO-A1-98/35975
- US-A- 5 576 425
- US-A- 5 681 938

## Description

La présente invention est relative à un procédé de préparation d'Alkyl Polyglycosides peu colorés (couleur inférieur à 1,5 vcs) impliquant des agents de neutralisation de type carbonate.

Les AlkylPolyGlycosides ou APG sont probablement les meilleurs exemples de tensioactifs biosourcés disponibles aujourd'hui sur le marché. Leurs structures moléculaires se caractérisent par la présence simultanée d'une tête hydrophile dérivée de sucres réducteurs (D-glucose,D-xylose ou D-rhamnose sont les sucres réducteurs principalement disponibles à l'échelle industrielle sur le marché) et d'une chaîne hydrocarbonée lipophile plus ou moins longue (cf. formule I : structure simplifiée d'un APG).

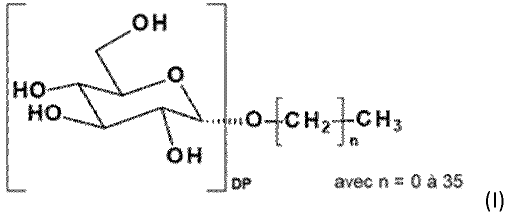

Leur procédé de fabrication à l'échelle industrielle est relativement simple et utilise comme matières premières i) le glucose, le xylose ou le rhamnose cristallisé issus respectivement de l'hydrolyse totale d'amidon de blé, de maïs ou de pomme de terre ou de l'hydrolyse d'hémicelluloses de bois et ii) des alcools gras provenant de la filière oléochimique (hydrogénation d'esters méthyliques issus de la transestérification de triglycérides végétaux). Les réactions de glycosylation de Fischer consistent alors à relier ces deux matières premières entre elles en créant une liaison chimique covalente, comme par exemple dans la réaction (II) entre le glucose et un alcool.

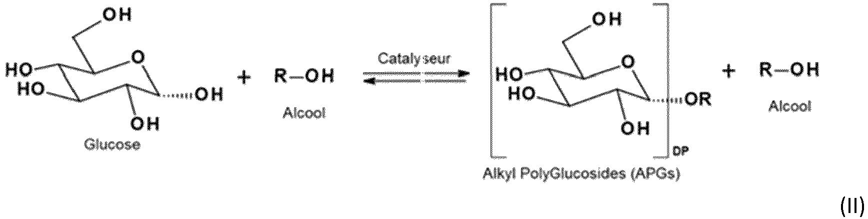

Pour réaliser cette réaction de glycosylation, un catalyseur acide d'origine minérale ou organique est nécessaire et un excès d'alcools est systématiquement introduit jouant ainsi le rôle de réactif et de solvant. En fin de réaction, les APG se retrouvent dispersés ou solubilisés dans l'excès d'alcools qui n'a pas réagi.

Les APG se distinguent par la nature, et la longueur de la chaîne alkyle hydrocarbonée R ainsi que par leur Degré de Polymérisation moyen DP supérieur à 1 mais inférieur ou égal à 2,5.

A l'issue de la phase réactionnelle de glycolysation, une étape de neutralisation est opérée afin de désactiver le catalyseur et de stopper la réaction.

Selon la longueur de la chaîne alkyle de l'alcool et l'utilisation associée, ledit alcool est soit éliminé, soit conservé.

L'étape de neutralisation diffère selon la longueur de la chaîne alkyle hydrocarbonée. Dans le cas où celle-ci présente un nombre d'atomes de carbone inférieur à 12, la neutralisation est réalisée par une solution aqueuse d'hydroxyde de sodium. L'excès d'alcools gras présent en fin de glycosylation est ensuite éliminé par distillation sous vide poussé ou distillation moléculaire, ou par évaporation, généralement, à l'aide d'un évaporateur couche mince à film tombant, ou d'un évaporateur couche mince à court trajet, et le concentré d'APG recueilli est finalement solubilisé dans l'eau. Les produits commerciaux ainsi obtenus se présentent donc sous la forme de solutions aqueuses d'APG avec une concentration massique comprise entre 40 et 80%.

Dans le cas où la chaîne alkyle hydrocarbonée R présente un nombre d'atome de carbone supérieur ou égale à 12, la neutralisation est généralement réalisée par l'hydroxyde de sodium ou par l'hydroxyde de potassium, seuls ou en combinaison avec un agent réducteur tel que décrit dans le Brevet Européen publié sous le numéro EP0077167, dans la demande de Brevet Européen publiée sous le numéro EP0338151 A1, dans le Brevet Européen EP0388857 B1, comme par exemple le borohydrure de sodium (NaBH₄) ou l'hypophosphite de sodium (NaH₂PO₂). Le mélange d'APG et de l'excès d'alcools gras est isolé après neutralisation et est commercialisé tel quel. La proportion massique des APG et des Alcools gras dépend de la stœchiométrie molaire retenue au départ pour les matières premières et de leur réactivité. Toutefois, des proportions de 5 à 30% massique d'APG et de 70 à 95% d'alcools gras sont généralement observées. Les compositions obtenues peuvent se présenter sous une forme solide, comme par exemple sous la forme d'écailles ou de perles, ou sous une forme liquide, en fonction de la nature de la chaîne alkyle hydrocarbonée R.

Cependant, l'étape de neutralisation des APG dont la chaîne alkyle hydrocarbonée R comporte un nombre d'atome de carbone supérieur ou égale à 12) par une base de l'état de la technique (exemple NaOH, KOH), pour atteindre une valeur du pH d'une dispersion à 5% massique dans l'eau du milieu neutralisé comprise entre 5,5 et 7,5, engendre une coloration importante du produit.

Par "mesure du pH d'une dispersion à 5% massique dans l'eau", on désigne au sens de la présente invention la méthode analytique de mesure de pH d'une dispersion d'une composition à base d'APG selon les dispositions de la norme NF EN 1262, ladite mesure est réalisée par mesure potentiométrique à l'aide d'une électrode pH (milieux aqueux) combinée et d'un pH mètre.

Cette coloration peut altérer les qualités organoleptiques des produits finis dans lesquels les compositions contenant les APG sont introduites. C'est pour cela que des solutions sont apportées pour minimiser la coloration des compositions contenant les APG dont la chaîne alkyle hydrocarbonée R comporte un nombre d'atome de carbone supérieur ou égale à 12. Deux leviers connus de l'état de la technique sont classiquement employés pour obtenir de telles compositions à base APG dont la chaîne alkyle hydrocarbonée R comporte un nombre d'atome de carbone supérieur ou égale à 12 peu colorées (< 1,5 vcs).

Par "composition peu colorée", on désigne au sens de la présente invention une composition dont l'indice de couleur Gardner, tel que défini par la norme DIN-ISO 4630, est inférieur ou égal à 1,5 VCS. L'indice de couleur Gardner est mesuré en utilisant un colorimètre LICO 200/Dr LANGE (ou équivalent) qui effectue des mesures par transmission de la lumière sur tout milieu. Un tel colorimètre fonctionne avec une lampe halogène correspondant à l'illuminant normalisé C, défini par la norme DIN 5033 et avec un observateur normalisé 2°. Pendant la mesure, un faisceau de rayonnement de référence compense les variations des valeurs enregistrées dues aux différences de lampe et de température.

Le premier levier consiste à associer un agent réducteur à la base utilisée. Parmi ces agents réducteurs, nous pouvons citer le borohydrure de sodium (NaBH₄) ou de l'hypophosphite de sodium (NaH₂PO₂). Cette solution n'est pas entièrement satisfaisante En effet, bien que très efficace pour minimiser la coloration de la composition traitée, le NaBH4 est un agent réducteur dangereux à manipuler et à mettre en œuvre (produit corrosif, dégagement d'hydrogène). Le NaH₂PO₂ est quant à lui très peu efficace même s'il est introduit à forte concentration.

Le second levier couramment utilisé et décrit dans l'état de la technique, pour minimiser la couleur des compositions à base d'APG, dont la chaîne alkyle hydrocarbonée R comporte un nombre d'atome de carbone supérieur ou égale à 12, est la réalisation d'une décoloration au péroxyde d'hydrogène (H₂O₂) lors de l'étape de finition. Bien qu'efficace, cette étape est néanmoins fastidieuse car elle nécessite d'ajuster la valeur du pH d'une dispersion à 5% massique dans l'eau entre 7,0 et 7,5 tout en maintenant le pouvoir oxydant du milieu par ajouts de d'H₂O₂. Cette étape délicate à réaliser peut durer plusieurs heures et donc accroître significativement la durée de production, en diminuant la productivité.

D'autres documents divulguent des procédés de préparation de l'art antérieur. Ainsi, EP0092876A1 décrit une préparation par transglycosylation comprenant une étape de neutralisation réalisée avec Na2CO3. US5681938A décrit un procédé de préparation d'APG comprenant une étape de réalisation réalisée avec une amine tertiaire. US5576425A divulgue une étape de neutralisation incluant des carbonates avec MgO comme base préférée. Enfin, WO98/35975A1 décrit une préparation d'APG comprenant l'utilisation d'un catalyseur sulfate binaire lui-même étant un mélange de H2SO4 et d'une base inorganique incluant les carbonates.Le problème technique à résoudre est donc de trouver une alternative à la neutralisation des compositions à base d'APG émulsionnants dont la chaîne alkyle hydrocarbonée R comporte un nombre d'atome de carbone supérieur ou égale à 12. Cette alternative doit être efficace et facile à mettre en œuvre, tout en garantissant une couleur inférieure ou égale à à 1,5 vcs sans mettre en œuvre une étape de décoloration. Une solution de la présente invention est un procédé de préparation d'une composition (C), de couleur inférieure ou égale à 1,5 vcs, comprenant pour 100% de sa masse :
(i) une quantité supérieure ou égale à 40% massique et inférieure ou égale à 95% massique d'un alcool de formule (I) :

   R-OH (I),

   dans laquelle R représente un radical hydrocarboné, linéaire ou ramifié, saturé ou insaturé, pouvant comporter au moins un fonction hydroxy, et comportant de douze à vingt-deux atomes de carbone, ou d'un mélange d'alcools de formule (I) ;
(ii) une quantité supérieure ou égale à 5% massique et inférieure ou égale à 60% massique d'une composition (C1) représentée par la formule (II) :

   R-O-(G)x-H (II),

   dans laquelle le reste G représente le reste d'un sucre réducteur, R représente un radical tel que défini dans la formule (I) et x, qui indique le degré moyen de polymérisation du reste G représente un nombre décimal supérieur à 1,05 et inférieur ou égal à 2,5, ou d'un mélange de compositions (C1) de formule (II) ;

étant entendu que la somme des proportions massiques des composés dans la composition (C) de formules (I) et (II) est égale à 100% massique ;
ledit procédé comprenant successivement :
   a) Une étape a) de glycosylation, consistant en une réaction entre au moins un alcool de formule (I) et au moins un sucre réducteur de formule (III) : H-O-(G)-H (III) , en présence d'au moins un catalyseur acide (CA), à une température supérieure ou égale à 100°C et inférieure ou égale à 120°C, le catalyseur acide (CA) étant choisi parmi les éléments du groupe constitué par l'acide sulfurique, l'acide chlorhydrique, l'acide phosphorique, l'acide nitrique, l'acide hypophosphoreux, l'acide méthane-sulfonique, l'acide para- toluène sulfonique, l'acide trifluorométhane sulfonique et les résines échangeuses d'ions acides,
   b) Une étape b) de neutralisation du milieu réactionnel issu de l'étape a) avec une solution aqueuse comprenant un agent basique (Ab) choisi parmi les éléments du groupe constitué par :
      ▪ les carbonates de formule (IVa) :

         XnCO₃ (IVa),

         dans laquelle X représente un atome de sodium ou de potassium et n est un nombre entier égal à 2, ou bien X représente un atome de calcium ou un atome de magnésium et n est un nombre entier égal à 1,
         ou
      ▪ les hydrogénocarbonates de formule (IVb) :

         Y(HCO₃)m (IVb),

         dans laquelle Y représente un atome de sodium ou de potassium et m est un nombre entier égal à 1, ou bien Y représente un atome de calcium ou un atome de magnésium et m est un nombre entier égal à 2,
      cette étape de neutralisation étant réalisée de manière à obtenir un milieu réactionnel dont une dispersion à 5% massique dudit milieu réactionnel dans l'eau présente une valeur de pH comprise entre 5,5 et 7,5,
   c) Une étape c) d'élimination du sucre de formule (III), qui n'a pas réagi à l'étape a), du milieu réactionnel neutralisé, et
   d) Une étape d) de récupération d'au moins une composition (C) de couleur inférieure ou égale à 1,5 vcs.

L'indice de couleur caractérisant la composition (C) préparée selon le procédé objet de la présente invention, est l'indice de couleur Gardner, tel que défini par la norme DIN-ISO 463. L'indice de couleur Gardner est mesuré en utilisant un colorimètre LICO 200/Dr LANGE (ou équivalent) qui effectue des mesures par transmission de la lumière sur tout milieu. Un tel colorimètre fonctionne avec une lampe halogène correspondant à l'illuminant normalisé C, défini par la norme DIN 5033 et avec un observateur normalisé 2°. Pendant la mesure, un faisceau de rayonnement de référence compense les variations des valeurs enregistrées dues aux différences de lampe et de température.

L'unité d'expression de l'indice de couleur Gardner, caractérisant la composition (C) préparée selon le procédé objet de la présente invention, est le VCS.

Selon le cas, le procédé selon l'invention peut présenter une ou plusieurs des caractéristiques suivantes :
- ladite composition (C1) consiste en un mélange de composés représentés par les formules (II1), (II2), (II3), (II4) et (II5):

   R-O-(G)1-H (II1),

   R-O-(G)2-H (II2),

   R-O-(G)3-H (II3),

   R-O-(G)4-H (II4),

   R-O-(G)5-H (II5),

   dans les proportions molaires respectives a1, a2, a3, a4 et a5, telles que:
   ▪ la somme: a1+ a2 + a3 + a4 + a5 est égale à 1, et
   ▪ la somme a1 + 2a2 + 3a3 + 4a4 + 5a5 est égale à x ;
- la composition (C) comprend une quantité inférieure ou égale à 2% massique du sucre réducteur de formule (III) :

   H-O-(G)-H (III) ;

   étant entendu que la somme des proportions massiques des composés de formules (I), (II) et (III) dans la composition (C) est égale à 100% massique ;
- l'agent basique (Ab) présent dans la solution aqueuse est le carbonate de sodium de formule (IVa) dans laquelle X représente l'atome de sodium et n est égal à 2 ;
- le procédé comprend après l'étape c) une étape d'ajustement complémentaire de la valeur du pH d'une dispersion à 5% massique dans l'eau du milieu réactionnel par ajout d'une solution aqueuse comprenant l'agent basique (Ab) tel que défini précédemment de manière à obtenir une valeur dudit pH comprise entre 5,5 et 7,5 ;
- le sucre réducteur de formule (III) choisi pour la glycolysation de l'étape a) est choisi parmi les éléments du groupe constitué par le glucose, le xylose, l'arabinose et le rhamnose ;
- l'étape c) d'élimination du sucre réducteur de formule (III) est réalisée par filtration, centrifugation ou décantation, ou par toute autre technique de séparation solide-liquide connue par l'homme du métier ; Lorsque cette étape d'élimination du sucre réducteur de formule (III) non réagi met en oeuvre une filtration, le milieu filtrant peut être du papier filtre de taille de pores adaptée (supérieur ou égal à 10 micromètres), ou bien une plaque filtrante de cellulose en présence optionnelle d'au moins un adjuvant de filtration connu de l'homme du métier ;
- A l'étape b) la solution aqueuse d'agent basique (Ab) comprend entre 10 % et 40 % massique dudit agent basique (Ab) ;
- l'étape a) comprend les sous-étapes successives suivantes :
   i) Introduction d'un alcool de formule (I) ou d'un mélange d'alcools de formule (I), dans un réacteur (Ré) équipé d'une agitation mécanique et d'un dispositif de mise sous vide ;
   ii) Chauffage de l'alcool de formule (I) à une température comprise entre 80°C et 90°C sous agitation mécanique;
   iii) Chargement du sucre réducteur de formule (III) dans le réacteur (Ré) ;
   iv) Introduction d'au moins un catalyseur acide dans le réacteur (Ré),
   v) Chauffage sous vide partiel du milieu réactionnel issu de la sous-étape iv) et présent dans le réacteur (Ré) à une température comprise entre 100°C et -110°C pendant la durée de la réaction, et
   vi) Refroidissement du milieu issu de la sous-étape v) à une température comprise entre 70°C et 80°C ;
- dans la formule (I) et/ou dans la formule (II), le radical R est choisi parmi les radicaux suivants : lauryle (ou n-dodécyle) myristyle (ou n-tétradécyle ), n-pentadécyle, cétyle (ou n-hexadécyle), n-heptadécyle, stéaryle (ou n-octadécyle), palmitoléyle (ou 9-hexadécényle), oléyle (ou 9-octadécényle), linoléyle (9,12-octadecadiényle), linolényle (ou 6,9,12-octadécatriényle) n-nonadécyle, arachidyle (ou n-eicosyle), béhényle (ou n-docosyle), érucyle (13-docosényle), ou 12-hydroxystéaryle ;
- dans la formule (I) et/ou dans la formule (II), le radical R est choisi parmi les radicaux suivants : 2-hexyl octyle, 2-hexyl décyle, 2-hexyl dodécyle, 2-octyl décyle, 2-octyl dodécyle, 2-décyl tétradécyle, isostéaryle (ou 16-méthyl heptadécyle) ou isomyristyle (ou 13-méthyl tridécyle) ;
- pendant les sous-étapes ii) à iv) le réacteur (Ré) est inerté sous azote ;
- Le procédé comprend entre les étapes ii) et iii) une étape de mise sous vide, de préférence à une pression inférieure ou égale à 50 millibars ;
- L'étape vi) est réalisée à pression atmosphérique.

L'emploi de carbonates de formule (IVa) ou d'hydrogénocarbonates de formule (IVb) ne contribue pas à augmenter la couleur de la composition (C) (la présence d'un agent réducteur n'étant pas alors nécessaire) tout en neutralisant au pH désiré d'une dispersion à 5% massique de la composition (C) (dont la valeur est comprise entre 5,5 et 7,5). L'utilisation d'un agent basique (Ab) permet d'éviter une étape de décoloration impliquant l'utilisation d'un agent péroxyde, ou autres, puisqu'elle permet d'atteindre une couleur inférieure ou égale à 1,5 vcs.

Par sucre réducteur, on désigne dans la définition de la formule (II) et dans la définition de la formule (III), les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990.

La structure oligomérique (G)x présente dans la formule (II), peut se présenter sous toutes formes d'isoméries, qu'il s'agisse d'isomérie optique, d'isomérie géométrique ou d'isomérie de position ; elle peut aussi représenter un mélange d'isomères.

Dans la formule (II) telle que définie ci-dessus, le radical R est lié à G par le carbone anomérique du reste saccharide, de manière à former une fonction acétal.

Selon un aspect particulier de la présente invention, dans la définition des composés de formules (II) et de formule (III), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le rhamnose, dextrane ou le tallose.

Selon un aspect particulier de la présente invention, dans la définition des composés de formule (II), G représente le reste d'un sucre réducteur choisi parmi les restes du glucose, du xylose, de l'arabinose ou du rhamnose, et x représente un nombre décimal supérieur ou égal à 1,05 et inférieur ou égal à 2,5.

Selon un aspect encore plus particulier de la présente invention, dans la définition des composés de formule (II), G représente le reste d'un sucre réducteur est choisi parmi les restes du glucose, du xylose, de l'arabinose ou du rhamnose, et x représente un nombre décimal supérieur ou égal à 1,05 et inférieur ou égal à 2,0, et encore plus particulièrement supérieur ou égal à 1,25 et inférieur ou égal à 2,0.

Selon un autre aspect particulier de la présente invention, le sucre réducteur de formule (III) est choisi parmi les éléments du groupe constitué par le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le rhamnose, dextrane ou le tallose.

Selon un aspect plus particulier de la présente invention, le sucre réducteur de formule (III) est choisi parmi le glucose, le xylose, l'arabinose ou le rhamnose.

Le procédé selon l'invention consiste à neutraliser le milieu en fin de réaction de glycosylation par ajout d'une solution aqueuse comprenant un agent basique (Ab) pour atteindre une valeur du pH d'une dispersion à 5% massique dudit milieu dans l'eau comprise entre 5,5 et 7,5. Le sucre réducteur de formule (III) résiduel est ensuite éliminé par filtration et, optionnellement, un nouvel ajout d'une solution aqueuse d'un agent basique (Ab) peut être réalisé si la valeur du pH de la dispersion à 5% massique dans l'eau de la composition (C) est inférieure à 5,5 (étape de finition).

Selon un aspect particulier, le procédé a pour objet la préparation d'une composition (C) de couleur inférieure ou égale à 1,5 VCS, comprenant pour 100% de sa masse :
- de 45% à 55% massique d'un mélange (M1) d'alcools de formule (I) comprenant pour 100% de la masse dudit mélange (M1), 50% massique d'un alcool de formule (I) dans laquelle R représente le radical n-hexadécyle et 50% massique d'un alcool de formule (I) dans laquelle R représente le radical n-octadécyle ;
- de 45% à 54% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle ;
- de 0% à 1% massique de glucose.

Selon un aspect particulier, le procédé a pour objet la préparation d'une composition (C) de couleur inférieure ou égale à 1,5 VCS, comprenant pour 100% de sa masse :
- de 45% à 55% massique d'un mélange (M'1) d'alcools de formule (I) comprenant pour 100% de la masse dudit mélange (M'1), 70% massique d'un alcool de formule (I) dans laquelle R représente le radical n-hexadécyle et 30% massique d'un alcool de formule (I) dans laquelle R représente le radical n-octadécyle ;
- de 45% à 54% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle ;
- de 0% à 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange (M"1) d'alcools de formule (I) comprenant pour 100% de la masse dudit mélange (M"1), 50% massique d'un alcool de formule (I) dans laquelle R représente le radical n-hexadécyle et 50% massique d'un alcool de formule (I) dans laquelle R représente le radical n-octadécyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle ;
- de 0% à 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange (M‴1) d'alcools de formule (I) comprenant pour 100% de la masse dudit mélange (M"'1), 70% massique d'un alcool de formule (I) dans laquelle R représente le radical n-hexadécyle et 30% massique d'un alcool de formule (I) dans laquelle R représente le radical n-octadécyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle ;
- de 0% à 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un alcool de formule (I) dans laquelle R représente le radical n-tétradécyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-tétradécyle ;
- de 0% à 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-dodécyle, le radical n-tétradécyle, le radical n-hexadécyle et le radical n-octadécyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical représente le radical n-dodécyle, le radical n-tétradécyle, le radical n-hexadécyle, et le radical n-octadécyle ;
- de 0% à 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-eicosyl et le radical n-docosyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-eicosyl et le radical n-docosyle ;
- de 0% à 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-dodécyle, le radical n-tétradécyle, le radical n-hexadécyle, le radical n-eicosyl et le radical n-docosyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-dodécyle, le radical n-tétradécyle, le radical n-hexadécyle, le radical n-eicosyl et le radical n-docosyle ;
- de 0% à 1% massique de glucose.

Selon un aspect particulier, le procédé a pour objet la préparation d'une composition (C) de couleur inférieure ou égale à 1,5 VCS, comprenant pour 100% de sa masse :
- de 70% à 90% massique d'un mélange d'alcools de formule (I) dans laquelle R représente le radical n-dodécyle, le radical n-tétradécyle, le radical n-hexadécyle, le radical n-eicosyl et le radical n-docosyle ;
- de 10% à 29% massique d'au moins une composition (C1) représentée par la formule (II) dans laquelle G représente le radical xylosyl ou α,β-D-xylopyranosyl, obtenu à partir de la suppression du groupe hydroxyl hémiacétal du α,β-D-xylopyranose, x représente un nombre décimal supérieur ou égal à 1,05 et inférieur ou égal à 2,0, R représente le radical 2-octyl dodécyle ;
- de 0% à 1% massique de xylose.

Selon un aspect particulier, l'agent basique (Ab) présent dans la solution aqueuse est le carbonate de potassium de formule (IVa) dans laquelle X représente l'atome de potassium et n est égal à 2.

Selon un aspect particulier, l'agent basique (Ab) présent dans la solution aqueuse est l'hydrogénocarbonate de sodium de formule (IVb) dans laquelle Y représente un atome de sodium et m est égal à 1.

Selon un aspect particulier, le catalyseur acide (CA) est choisi parmi les éléments du groupe constitué par l'acide sulfurique, l'acide phosphorique, l'acide hypophosphoreux, l'acide méthane-sulfonique, l'acide p-toluène sulfonique.

### Exemples

### A- Comparaison de l'effet de l'agent neutralisant sur la couleur d'une composition d'alcools gras et d'alkylpolyglucosides lorsque l'agent basique neutralisant utilisé est un carbonate selon l'invention ou la soude ( agent neutralisant comparatif).

Des comparaisons entre un carbonate et la soude comme agent neutralisant ont été effectuées. Pour ceci, des réactions de glycosylations ont été menées à partir de glucose cristallisé et de différents alcools gras sous forme de coupes ou purs : coupe cétéarylique C-16/18, coupe arachydilique/béhénique C-20/22, et dodécanol-1 (alcool C-12).

Le tableau 1 suivant rassemble des résultats de neutralisation mettant en jeu le carbonate de sodium Na₂CO₃ (neutralisant du procédé selonl'invention), d'une part, et la soude NaOH, d'autre part (neutralisant du procédé comparatif).

### 1- Exemples selon l'invention

### Exemple 1.1 (Coupe d'alcools 16/18 et Na₂CO₃ comme agent neutralisant) selon l'invention

### ETAPE 1 : Réaction de Glycosylation :

967,4 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 Torrs. Une quantité de glucose anhydre sous forme de poudre est ajoutée de sorte à ce que le rapport molaire entre les alcools gras et le glucose soit de 6/1. Le milieu est inerté sous azote. Pour démarrer la réaction d'éthérification 0,9 g d'une solution aqueuse de H₃PO₂ à 50 % puis 1,1 g d'une solution aqueuse de H₂SO₄ à 98 % sont ajoutés et la température est augmentée et maintenue à 105°C. La réaction est poursuivie pendant une durée de 5h45.

### ETAPE 2 : Neutralisation du milieu réactionnel :

Le milieu est ensuite refroidi à 80°C à pression atmosphérique puis neutralisé en introduisant 4,61 g d'une solution aqueuse de Na₂CO₃ à 25 %. Le produit est ensuite introduit dans un flacon en verre et placé à l'étuve à 80°C pendant 24 heures afin de décanter le glucose résiduel. Le produit (phase supérieure) est récupéré et référencé (Composition 1).

### Analyses :

- La valeur du pH d'une dispersion à 5% massique dans l'eau de la Composition 1 est de 5,5, et
- la mesure de la couleur de Composition 1 est de 0,7 VCS.

### Exemple 1.2 (Coupe d'alcools 20/22 et Na₂CO₃ comme agent neutralisant)

### ETAPE 1 : Réaction de Glycosylation :

240,5 g d'un mélange d'alcool arachidylique (alcool C20) et d'alcool bénéhylique/arachidylique (alcool C-220/22), dans un ratio massique alcool C20/alcool C22 de 70/30, sont chargés dans un réacteur équipé d'une agitation mécanique et d'un montage de distillation sous vide. Le mélange d'alcool est fondu à 85°C et mis sous agitation et sous barbotage d'azote. Le milieu est mis sous vide à des pressions inférieures à 50 Torrs. 24,6 g de glucose dextrose anhydre sous forme de poudre sont ajoutés. Le milieu est inerté sous azote. Pour démarrer la réaction d'éthérification 0,2 g d'une solution aqueuse de H₃PO₂ à 50 % puis 0,4 g d'une solution aqueuse de H₂SO₄ à 98 % sont ajoutés et la température est augmentée et maintenue à 105°C. La réaction est poursuivie pendant 4h30.

### ETAPE 2 : Neutralisation du milieu réactionnel :

Le milieu est ensuite refroidi à 80°C à pression atmosphérique puis neutralisé en introduisant 3,3 g d'une solution aqueuse de Na₂CO₃ à 10 %. Le produit possède un pH de 5,6 et une couleur de 0,3 VCS. Le produit est ensuite introduit dans un flacon en verre et placé à l'étuve à 80°C pendant 24 heures afin de décanter le glucose dextrose résiduel. Le produit (phase supérieure) possède un pH de 4,8 et une couleur de 0,3 VCS. Une étape de finition est réalisée à 80°C en introduisant 0,19 g d'une solution aqueuse de Na₂CO₃ à 10 % pour obtenir un produit final (Composition 2).

### Analyses :

- La valeur du pH d'une dispersion à 5% massique dans l'eau de la Composition 2 est de 7,0, et
- la mesure de la couleur de la Composition 2 est de 0,6 VCS.

### Exemple 1.3 (alcool C12 et Na₂CO₃ comme agent neutralisant)

Le mode opératoire de l'exemple 1.1 est reproduit en substituant les 967,4 g d'alcool cétéarylique par 414,5 grammes de dodécanol-1, et en utilisant une quantité de glucose anhydre sous forme de poudre de sorte à ce que le rapport molaire entre le dodécanol-1 et le glucose soit de 6/1.

On obtient ainsi le produit référencé (Composition 3).

### ETAPE 1 : Réaction de Glycosylation :

131,3 g de dodécanol-1 (alcool C-12) sont chargés dans un réacteur équipé d'une agitation mécanique et d'un montage de distillation sous vide. L'alcool est placé à 85°C et mis sous agitation et sous barbotage d'azote. Le milieu est mis sous vide à 30 Torrs. 18,3 g de glucose anhydre sous forme de poudre sont ajoutés. Le milieu est inerté sous azote. Pour démarrer la réaction d'éthérification 0,14 g d'une solution aqueuse de H₃PO₂ à 50 % puis 0,23 g d'une solution aqueuse de H₂SO₄ à 98 % sont ajoutés et la température est augmentée et maintenue à 105°C. La réaction est poursuivie pendant 5h00.

### ETAPE 2 : Neutralisation du milieu réactionnel :

Le milieu est ensuite refroidi à 67°C à pression atmosphérique puis neutralisé en introduisant 0,29 g d'une solution aqueuse de Na₂CO₃ à 25 % sous agitation. Le produit est filtré sur papier filtre (^{~} 10 µm) afin d'éliminer le glucose résiduel pour obtenir le produit référencé (Composition 3).

### Analyses :

- La valeur du pH d'une dispersion à 5% massique dans l'eau de la Composition 3 est de 6,1, et
- la mesure de la couleur de la Composition 3 est de 1,5 VCS.

### Exemple 1.4 (Coupe d'alcools 16/18 et Na₂CO₃ comme agent neutralisant)

Le mode opératoire de l'exemple 1.1 est reproduit en substituant les 4,61 grammes d'une solution aqueuse de Na₂CO₃ à 25 % par la masse adaptée de ladite solution de Na₂CO₃ à 25 % de sorte à obtenir une valeur du pH de 7,4 pour une dispersion à 5% de la composition finale (Composition 4) ainsi obtenue.

### Analyses :

- La valeur du pH d'une dispersion à 5% massique dans l'eau de la Composition 4 est de 7,4, et
- la mesure de la couleur de la Composition 4 est de 1,0 VCS.

### Exemple 1.5 (Coupe d'alcools 16/18 et Na₂CO₃ comme agent neutralisant)

Le mode opératoire de l'exemple 1.1 est reproduit en substituant les 4,61 grammes d'une solution aqueuse de Na₂CO₃ à 25 % par la masse adaptée de ladite solution de Na₂CO₃ à 25 % de sorte à obtenir une valeur du pH de 8,2 pour une dispersion à 5% de la composition finale (Composition 5) ainsi obtenue.

On obtient ainsi le produit référencé (Composition 5).

### Analyses :

- La valeur du pH d'une dispersion à 5% massique dans l'eau de la Composition 5 est de 8,2, et
- la mesure de la couleur de la Composition 5 est de 2,3 VCS.

### 2- Exemples comparatifs

### Exemple 2.1 (Coupe d'alcools 16/18 et NaOH comme agent neutralisant)

### ETAPE 1 : Réaction de Glycosylation :

240,9 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 Torrs. 31,6 g de glucose anhydre sous forme de poudre sont ajoutés. Le milieu est inerté sous azote. Pour démarrer la réaction d'éthérification 0,22 g d'une solution aqueuse de H₃PO₂ à 50 % puis 0,28 g d'une solution aqueuse de H₂SO₄ à 98 % sont ajoutés et la température est augmentée et maintenue à 105°C. La réaction est poursuivie pendant 5h45.

### ETAPE 2 : Neutralisation du milieu réactionnel :

Le milieu est ensuite refroidi à 72°C à pression atmosphérique puis neutralisé en introduisant 0,73 g d'une solution aqueuse de NaOH à 25 % sous agitation. Le produit est ensuite introduit dans un flacon en verre et placé à l'étuve à 80°C pendant 24 heures afin de décanter le glucose résiduel. Le produit (phase supérieure) est récupéré **(Composition Comparative 1).**

### Analyses :

- La valeur du pH d'une dispersion à 5% massique dans l'eau de la Composition Comparative 1 est de 6,1, et
- la mesure de la couleur de la Composition Comparative 1 est de 4,1 VCS.

### Exemple 2.2 (Coupe d'alcools 20/22 et NaOH comme agent neutralisant)

Le mode opératoire de l'exemple 2.1 est reproduit en substituant les 240,9 g d'alcool cétéarylique par 59,9 grammes d'un mélange d'alcool arachydilique (C20) et d'alcool bénéhylique (C22) dans un ratio massique (alcool C20/alcool C22) de 70/30, et en utilisant une quantité de glucose anhydre sous forme de poudre de sorte à ce que le rapport molaire entre les alcools gras et le glucose soit de 6/1.

### On obtient ainsi le produit référencé (Composition Comparative 2)

### Analyses :

- La valeur du pH d'une dispersion à 5% massique dans l'eau de la Composition Comparative 2 est de 5,7, et
- la mesure de la couleur de la Composition Comparative 2 est de 6,3 VCS.

### Exemple 2.3 (alcool C₁₂ et NaOH comme agent neutralisant)

### ETAPE 1 : Réaction de Glycosylation :

131,3 g de dodécanol-1 (C-12) sont chargés dans un réacteur équipé d'une agitation mécanique et d'un montage de distillation sous vide. Le dodécanol-1 est introduit à 85°C et mis sous agitation et sous barbotage d'azote. Le milieu est mis sous vide à 30 Torrs. 18,3 g de glucose anhydre sous forme de poudre sont ajoutés. Le milieu est inerté sous azote. Pour démarrer la réaction d'éthérification 0,14 g d'une solution aqueuse de H₃PO₂ à 50 % puis 0,23 g d'une solution aqueuse de H₂SO₄ à 98 % sont ajoutés et la température est augmentée et maintenue à 105°C. La réaction est poursuivie pendant 5h00.

### ETAPE 2 : Neutralisation du milieu réactionnel :

Le milieu est ensuite refroidi à 67°C à pression atmosphérique puis neutralisé en introduisant 0,29 g d'une solution aqueuse de NaOH à 25 % sous agitation. Le produit est filtré sur papier filtre (^{~} 10 µm) afin d'éliminer le glucose résiduel et d'obtenir le produit final référencé (Composition Comparative 3).

### Analyses :

- La valeur du pH d'une dispersion à 5% massique dans l'eau de la Composition Comparative 3 est de 6,0, et
- la mesure de la couleur de la Composition Comparative 3 est de 1,8 VCS.

**[Table 1]**

| Référence | Composition comparative 1 | Composition 1 | Composition comparative 2 | Composition 2 | Composition comparative 3 | Composition 3 |
|---|---|---|---|---|---|---|
| Sucre APG | Glucose | | | | | |
| Chaîne alkyle APG | C-16/18 | | C-20/22 | | C-12 | |
| Agent basique neutralisant | NaOH | Na₂CO₃ | NaOH | Na₂CO₃ | NaOH | Na₂CO₃ |
| pH 5% | 6,1 | 5,5 | 5,7 | 7,0 | 6,0 | 6,1 |
| Couleur (vcs) | 4,1 | 0,7 | 6,3 | 0,6 | 1,8 | 1,5 |

### Analyses et observations

Quelle que soit la longueur de chaîne grasse étudiée (de C-12 à C-22), l'utilisation d'une solution de NaOH comme agent basique neutralisant du milieu réactionnel, pour atteindre des valeurs de pH d'une dispersion à 5% massique dans l'eau supérieure ou égale à 5,5 dudit milieu réactionnel, colore fortement les compositions comprenant des alcools gras résiduels et les alkylpolyglucosides formés. En effet, la couleur mesurée pour ces essais oscille entre 1,8 et 6,3 vcs après filtration du sucre résiduel. En comparaison, lorsque l'étape de neutralisation est menée par une solution de Na₂CO₃ pour obtenir des valeurs de pH d'une dispersion à 5% massique dans l'eau supérieure ou égale à 5,5 dudit milieu réactionnel, on observe que les couleurs des compositions comprenant des alcools gras résiduels et les alkylpolyglucosides formés montrent des valeurs inférieures ou égales à 1,5 vcs.

### B- Influence de la valeur du pH d'une dispersion à 5% massique dans l'eau d'une composition d'alcools gras et d'alkylpolyglucosides sur la couleur du produit final, lorsque la phase de neutralisation est effectuée avec un carbonate

Trois essais expérimentaux ont été réalisés par la mise en œuvre du procédé général présenté précédemment. Ces trois essais (exemple 1.1, exemple 1.4 et exemple 1.5) décrivent la préparation de compositions comprenant l'alcool cétéarylique et des alkylpolyglucosides sur chaînes linéaires C16 et C18, lesdites compositions (Composition 1, Composition 4 et Composition 5) ayant été préparées par la mise en œuvre du procédé selon l'invention et se caractérisent par différentes valeurs de pH de la dispersion à 5% de chaque composition (*cf* tableau 2).

**[Table 2]**

| Essai | (Composition 1) | Composition 4) | (Composition 5) |
|---|---|---|---|
| pH(5%) | 6,4 | 7,4 | 8,2 |
| Couleur (VCS) | 0,7 | 1,0 | 2,3 |

### Observations et analyses

Les résultats obtenus montrent que lorsque la valeur du pH d'une dispersion à 5% massique de la composition préparée, est comprise entre 5,5 et 7,5, le procédé selon l'invention permet d'atteindre une valeur de la couleur inférieure ou égale à 1,5 VCS. En revanche, lorsque la valeur du pH d'une dispersion à 5% massique de la composition préparée, est de 8,2, la couleur de la composition obtenue est supérieure à la couleur maximale souhaitée de 1,5 VCS.

## Revendications

1. Procédé de préparation d'une composition (C), de couleur inférieure ou égale à 1,5 vcs, comprenant pour 100% de sa masse :
i) une quantité supérieure ou égale à 40% massique et inférieure ou égale à 95% massique d'un alcool de formule (I) :
R-OH (I),
dans laquelle R représente un radical hydrocarboné, linéaire ou ramifié, saturé ou insaturé, pouvant comporter au moins un fonction hydroxy, et comportant de douze à vingt-deux atomes de carbone, ou d'un mélange d'alcools de formule (I) ;
ii) une quantité supérieure ou égale à 5% massique et inférieure ou égale à 60% massique d'une composition (C1) représentée par la formule (II) :
R-O-(G)x-H (II),
dans laquelle le reste G représente le reste d'un sucre réducteur, R représente un radical tel que défini dans la formule (I) et x, qui indique le degré moyen de polymérisation du reste G représente un nombre décimal supérieur à 1,05 et inférieur ou égal à 2,5, ou d'un mélange de compositions (C1) de formule (II) ;
étant entendu que la somme des proportions massiques des composés de formules (I) et (II) dans la composition (C) est égale à 100% massique ;
ledit procédé comprenant successivement :
a) Une étape a) de glycosylation, consistant en une réaction entre au moins un alcool de formule (I) et au moins un sucre réducteur de formule (III) : H-O-(G)-H (III) , en présence d'au moins un catalyseur acide (CA), à une température supérieure ou égale à 100°C et inférieure ou égale à 120°C, le au moins un catalyseur acide (CA) étant choisi parmi les éléments du groupe constitué par l'acide sulfurique, l'acide chlorhydrique, l'acide phosphorique, l'acide nitrique, l'acide hypophosphoreux, l'acide méthane-sulfonique, l'acide para-toluène sulfonique, l'acide trifluorométhane sulfonique et les résines échangeuses d'ions acides,
b) Une étape b) de neutralisation du milieu réactionnel issu de l'étape a) avec une solution aqueuse comprenant un agent basique (Ab) choisi parmi les éléments du groupe constitué par :
▪ les carbonates de formule (IVa) :
XnCO₃ (IVa),
dans laquelle X représente un atome de sodium ou de potassium et n est un nombre entier égal à 2, ou bien X représente un atome de calcium ou un atome de magnésium et n est un nombre entier égal à 1,
ou
▪ les hydrogénocarbonates de formule (IVb) :
Y(HCO₃)m (IVb),
dans laquelle Y représente un atome de sodium ou de potassium et m est un nombre entier égal à 1, ou bien Y représente un atome de calcium ou un atome de magnésium et m est un nombre entier égal à 2,
la neutralisation étant réalisée de manière à obtenir un milieu réactionnel dont une dispersion à 5% massique dudit milieu réactionnel dans l'eau présente une valeur de pH comprise entre 5,5 et 7,5,
c) Une étape c) d'élimination du sucre de formule (III), qui n'a pas réagi à l'étape a), du milieu réactionnel neutralisé, et
d) Une étape d) de récupération d'au moins une composition (C) de couleur inférieure ou égale à 1,5 vcs.

2. Procédé selon la revendication 1, **caractérisé en ce que** ladite composition (C1) consiste en un mélange de composés représentés par les formules (II1), (II2), (II3), (II4) et (II5):
R-O-(G)1-H (II1),
R-O-(G)2-H (II2),
R-O-(G)3-H (II3),
R-O-(G)4-H (II4),
R-O-(G)5-H (II5),
dans les proportions molaires respectives a1, a2, a3, a4 et a5, telles que:
▪ la somme: a1+ a2 + a3 + a4 + a5 est égale à 1, et
▪ la somme a1 + 2a2 + 3a3 + 4a4 + 5a5 est égale à x.

3. Procédé selon l'une des revendications 1 ou 2, **caractérisé en ce que** la composition (C) comprend une quantité inférieure ou égale à 2% massique du sucre réducteur de formule (III) :
H-O-(G)-H (III) ;
étant entendu que la somme des proportions massiques des composés de formules (I), (II) et (III) dans la composition (C) est égale à 100% massique.

4. Procédé selon l'une des revendications 1 à 3, **caractérisé en ce que** l'agent basique (Ab) présent dans la solution aqueuse est le carbonate de sodium de formule (IVa) dans laquelle X est l'atome de sodium et n est égal à 2.

5. Procédé selon l'une des revendications 1 à 4, **caractérisé en ce qu'**il comprend après l'étape c) une étape d'ajustement complémentaire de la valeur du pH d'une dispersion à 5% massique dans l'eau du milieu réactionnel par ajout d'une solution aqueuse comprenant l'agent basique (Ab) tel que défini précédemment de manière à obtenir une valeur dudit pH comprise entre 5,5 et 7,5.

6. Procédé selon l'une des revendications 1 à 5, **caractérisé en ce que** le sucre réducteur de formule (III) choisi pour la glycolysation de l'étape a) est choisi parmi les éléments du groupe constitué par le glucose, le xylose, l'arabinose et le rhamnose .

7. Procédé selon l'une des revendications 1 à 6, **caractérisé en ce que** l'étape c) d'élimination du sucre réducteur de formule (III) est réalisée par filtration, centrifugation ou décantation.

8. Procédé selon l'une des revendications 1 à 7, **caractérisé en ce qu'**à l'étape b) la solution aqueuse d'agent basique (Ab) comprend entre 10% et 40% massique dudit agent basique (Ab).

9. Procédé selon l'une des revendications 1 à 8, **caractérisé en ce que** l'étape a) comprend les sous-étapes successives suivantes :
i) Introduction d'un alcool de formule (I) ou d'un mélange d'alcools de formule (I), dans un réacteur (Ré) équipé d'une agitation mécanique et d'un dispositif de mise sous vide ;
ii) Chauffage de l'alcool de formule (I) à une température comprise entre 80°C et 90°C sous agitation mécanique;
iii) Chargement du sucre réducteur de formule (III) dans le réacteur (Ré) ;
iv) Introduction d'au moins un catalyseur acide dans le réacteur (Ré),
v) Chauffage sous vide partiel du milieu réactionnel issu de la sous-étape iv) et présent dans le réacteur (Ré) à une température comprise entre 100°C et 110°C pendant la durée de la réaction, et
vi) Refroidissement du milieu issu de la sous-étape v) à une température comprise entre 70°C et 80°C.

10. Procédé selon la revendication 9, **caractérisé en ce que** dans la formule (I) et/ou dans la formule (II), le radical R est choisi parmi les radicaux suivants : lauryle (ou n-dodécyle) myristyle (ou n-tétradécyle ), n-pentadécyle, cétyle (ou n-hexadécyle), n-heptadécyle, stéaryle (ou n-octadécyle), palmitoléyle (ou 9-hexadécényle), oléyle (ou 9-octadécényle), linoléyle (9,12-octadecadiényle), linolényle (ou 6,9,12-octadécatriényle) n-nonadécyle, arachidyle (ou n-eicosyle), béhényle (ou n-docosyle), érucyle (13-docosényle), ou 12-hydroxystéaryle.

11. Procédé selon la revendication 9, **caractérisé en ce que** dans la formule (I) et/ou dans la formule (II), le radical R est choisi parmi les radicaux suivants : 2-hexyl octyle, 2-hexyl décyle, 2-hexyl dodécyle, 2-octyl décyle, 2-octyl dodécyle,, 2-décyl tétradécyle, isostéaryle (ou 16-méthyl heptadécyle) ou isomyristyle (ou 13-méthyl tridécyle).

12. Procédé selon l'une des revendications 9 à 11, **caractérisé en ce que** pendant les sous-étapes ii) à iv) le réacteur (Ré) est inerté sous azote.

13. Procédé selon l'une des revendications 9 à 12, **caractérisé en ce qu'**il comprend entre les sous-étapes ii) et iii) une sous-étape de mise sous vide, de préférence à une pression inférieure ou égale à 50 millibars.

14. Procédé selon l'une des revendications 9 à 13, **caractérisé en ce que** l'étape vi) est réalisée à pression atmosphérique.

## Patentansprüche

1. Ein Verfahren zur Herstellung einer Zusammensetzung (C) mit einer Farbe von höchstens 1,5 VCS, umfassend, bezogen auf 100% ihrer Masse:
i) eine Menge von mindestens 40 Massen-% und höchstens 95 Massen-% eines Alkohols der Formel (I):
R-OH (I),
wobei R einen Kohlenwasserstoffrest, linear oder verzweigt, gesättigt oder ungesättigt, darstellt, der mindestens eine Hydroxyfunktion enthalten kann und zwölf bis zweiundzwanzig Kohlenstoffatome aufweist, oder eine Mischung von Alkoholen der Formel (I);
ii) eine Menge von mindestens 5 Massen-% und höchstens 60 Massen-% einer Zusammensetzung (C1), dargestellt durch die Formel (II):
R-O-(G)x-H (II),
wobei der Rest G den Rest eines reduzierenden Zuckers darstellt, R einen Rest wie in Formel (I) definiert darstellt und x, der den mittleren Polymerisationsgrad des Restes G angibt, eine Dezimalzahl größer oder gleich 1,05 und kleiner oder gleich 2,5 darstellt, oder eine Mischung von Zusammensetzungen (C1) der Formel (II);
vorausgesetzt, dass die Summe der Massenanteile der Verbindungen der Formeln (I) und (II) in der Zusammensetzung (C) gleich 100 Massen-% ist;
wobei das Verfahren nacheinander umfasst:
a) Einen Schritt a) der Glykosylierung, bestehend aus einer Reaktion zwischen mindestens einem Alkohol der Formel (I) und mindestens einem reduzierenden Zucker der Formel (III):
H-O-(G)-H (III),
in Gegenwart von mindestens einem sauren Katalysator (CA), bei einer Temperatur von mindestens 100°C und höchstens 120°C, wobei der mindestens eine saure Katalysator (CA) ausgewählt ist aus den Elementen der Gruppe bestehend aus Schwefelsäure, Salzsäure, Phosphorsäure, Salpetersäure, hypophosphoriger Säure, Methansulfonsäure, p-Toluolsulfonsäure, Trifluormethansulfonsäure und sauren Ionenaustauscherharzen,
b) Einen Schritt b) der Neutralisation des aus Schritt a) resultierenden Reaktionsmediums mit einer wässrigen Lösung, umfassend ein basisches Mittel (Ab), ausgewählt aus den Elementen der Gruppe bestehend aus:
- Carbonaten der Formel (IVa): XnCO₃ (IVa), wobei X ein Natrium- oder Kaliumatom darstellt und n eine ganze Zahl gleich 2 ist, oder X ein Calcium- oder Magnesiumatom darstellt und n eine ganze Zahl gleich 1 ist, oder
- Hydrogencarbonaten der Formel (IVb): Y(HCO₃)m (IVb), wobei Y ein Natrium- oder Kaliumatom darstellt und m eine ganze Zahl gleich 1 ist, oder Y ein Calcium- oder Magnesiumatom darstellt und m eine ganze Zahl gleich 2 ist,
wobei die Neutralisation so durchgeführt wird, dass ein Reaktionsmedium erhalten wird, dessen 5-mass-%ige Dispersion in Wasser einen pH-Wert zwischen 5,5 und 7,5 aufweist,
c) Einen Schritt c) der Eliminierung des Zuckers der Formel (III), der in Schritt a) nicht reagiert hat, aus dem neutralisierten Reaktionsmedium, und
d) Einen Schritt d) der Rückgewinnung mindestens einer Zusammensetzung (C) mit einer Farbe von höchstens 1,5 VCS.

2. Verfahren nach Anspruch 1, **dadurch gekennzeichnet, dass** die Zusammensetzung (C1) aus einer Mischung von Verbindungen besteht, die durch die Formeln (II1), (II2), (II3), (II4) und (II5) dargestellt werden:
R-O-(G)1-H (II1),
R-O-(G)2-H (II2),
R-O-(G)3-H (II3),
R-O-(G)4-H (II4),
R-O-(G)5-H (II5),
in den jeweiligen molaren Anteilen a1, a2, a3, a4 und a5, so dass:
- die Summe: a1+a2 + a3 + a4 + a5 gleich 1 ist, und
- die Summe a1 + 2a2 + 3a3 + 4a4 + 5a5 gleich x ist.

3. Verfahren nach einem der Ansprüche 1 oder 2, **dadurch gekennzeichnet, dass** die Zusammensetzung (C) eine Menge von höchstens 2 Massen-% des reduzierenden Zuckers der Formel (III) umfasst:
H-O-(G)-H (III);
vorausgesetzt, dass die Summe der Massenanteile der Verbindungen der Formeln (I), (II) und (III) in der Zusammensetzung (C) gleich 100 Massen-% ist.

4. Verfahren nach einem der Ansprüche 1 bis 3, **dadurch gekennzeichnet, dass** das in der wässrigen Lösung vorhandene basische Mittel (Ab) Natriumcarbonat der Formel (IVa) ist, wobei X das Natriumatom darstellt und n gleich 2 ist.

5. Verfahren nach einem der Ansprüche 1 bis 4, **dadurch gekennzeichnet, dass** es nach Schritt c) einen zusätzlichen Schritt zur Einstellung des pH-Wertes einer 5-mass-%igen Dispersion des Reaktionsmediums in Wasser durch Zugabe einer wässrigen Lösung, umfassend das wie zuvor definierte basische Mittel (Ab), umfasst, um einen pH-Wert zwischen 5,5 und 7,5 zu erhalten.

6. Verfahren nach einem der Ansprüche 1 bis 5, **dadurch gekennzeichnet, dass** der für die Glykosylierung von Schritt a) ausgewählte reduzierende Zucker der Formel (III) ausgewählt ist aus den Elementen der Gruppe bestehend aus Glucose, Xylose, Arabinose und Rhamnose.

7. Verfahren nach einem der Ansprüche 1 bis 6, **dadurch gekennzeichnet, dass** Schritt c) der Eliminierung des reduzierenden Zuckers der Formel (III) durch Filtration, Zentrifugation oder Dekantierung durchgeführt wird.

8. Verfahren nach einem der Ansprüche 1 bis 7, **dadurch gekennzeichnet, dass** in Schritt b) die wässrige Lösung des basischen Mittels (Ab) zwischen 10 Massen-% und 40 Massen-% des besagten basischen Mittels (Ab) umfasst.

9. Verfahren nach einem der Ansprüche 1 bis 8, **dadurch gekennzeichnet, dass** Schritt a) die folgenden aufeinanderfolgenden Unterschritte umfasst:
i) Einführung eines Alkohols der Formel (I) oder einer Mischung von Alkoholen der Formel (I) in einen Reaktor (Ré), der mit einer mechanischen Rührung und einer Vakuumeinrichtung ausgestattet ist;
ii) Erhitzen des Alkohols der Formel (I) auf eine Temperatur zwischen 80°C und 90°C unter mechanischer Rührung;
iii) Beschickung des reduzierenden Zuckers der Formel (III) in den Reaktor (Ré);
iv) Einführung mindestens eines sauren Katalysators in den Reaktor (Ré),
v) Erhitzen des aus Unterschritt iv) resultierenden Reaktionsmediums, das sich in dem Reaktor (Ré) befindet, unter Teilvakuum auf eine Temperatur zwischen 100°C und 110°C für die Dauer der Reaktion, und
vi) Abkühlen des aus Unterschritt v) resultierenden Mediums auf eine Temperatur zwischen 70°C und 80°C.

10. Verfahren nach Anspruch 9, **dadurch gekennzeichnet, dass** in der Formel (I) und/oder in der Formel (II) der Rest R ausgewählt ist aus den folgenden Resten: Lauryl (oder n-Dodecyl), Myristyl (oder n-Tetradecyl), n-Pentadecyl, Cetyl (oder n-Hexadecyl), n-Heptadecyl, Stearyl (oder n-Octadecyl), Palmitoleyl (oder 9-Hexadecenyl), Oleyl (oder 9-Octadecenyl), Linoleyl (9,12-Octadecadienyl), Linolenyl (oder 6,9,12-Octadecatrienyl), n-Nonadecyl, Arachidyl (oder n-Eicosyl), Behenyl (oder n-Docosyl), Erucyl (13-Docosenyl), oder 12-Hydroxystearyl.

11. Verfahren nach Anspruch 9, **dadurch gekennzeichnet, dass** in der Formel (I) und/oder in der Formel (II) der Rest R ausgewählt ist aus den folgenden Resten: 2-Hexyloctyl, 2-Hexyldecyl, 2-Hexyldodecyl, 2-Octyldecyl, 2-Octyldodecyl, 2-Decyltetradecyl, Isostearyl (oder 16-Methylheptadecyl) oder Isomyristyl (oder 13-Methyltridecyl).

12. Verfahren nach einem der Ansprüche 9 bis 11, **dadurch gekennzeichnet, dass** der Reaktor (Ré) während der Unterschritte ii) bis iv) unter Stickstoff inertisiert wird.

13. Verfahren nach einem der Ansprüche 9 bis 12, **dadurch gekennzeichnet, dass** es zwischen den Unterschritten ii) und iii) einen Unterschritt der Vakuumanwendung umfasst, vorzugsweise bei einem Druck von höchstens 50 Millibar.

14. Verfahren nach einem der Ansprüche 9 bis 13, **dadurch gekennzeichnet, dass** Schritt vi) bei atmosphärischem Druck durchgeführt wird.

## Claims

1. A process for preparing a composition (C), having a color equal to or less than 1.5 vcs, comprising for 100% of its weight:
i) a quantity greater than or equal to 40% by weight and less than or equal to 95% by weight of an alcohol of formula (I):
R-OH (I),
in which R represents a hydrocarbon radical, linear or branched, saturated or unsaturated, possibly comprising at least one hydroxyl function, and comprising from twelve to twenty-two carbon atoms, or a mixture of alcohols of formula (I);
ii) a quantity greater than or equal to 5% by weight and less than or equal to 60% by weight of a composition (C1) represented by formula (II):
R-O-(G)x-H (II),
in which the residue G represents the residue of a reducing sugar, R represents a radical as defined in formula (I) and x, which indicates the average degree of polymerization of the residue G represents a decimal number greater than or equal to 1.05 and less than or equal to 2.5, or a mixture of compositions (C1) of formula (II);
provided that the sum of the weight proportions of the compounds of formulas (I) and (II) in composition (C) is equal to 100% by weight;
said process successively comprising:
a) A step a) of glycosylation, consisting of a reaction between at least one alcohol of formula (I) and at least one reducing sugar of formula (III):
H-O-(G)-H (III),
in the presence of at least one acid catalyst (CA), at a temperature greater than or equal to 100°C and less than or equal to 120°C, the at least one acid catalyst (CA) being chosen from the elements of the group consisting of sulfuric acid, hydrochloric acid, phosphoric acid, nitric acid, hypophosphorous acid, methane-sulfonic acid, para-toluene sulfonic acid, trifluoromethane sulfonic acid and acidic ion exchange resins,
b) A step b) of neutralizing the reaction medium resulting from step a) with an aqueous solution comprising a basic agent (Ab) chosen from the elements of the group consisting of:
- carbonates of formula (IVa): XnCO₃ (IVa), in which X represents a sodium or potassium atom and n is an integer equal to 2, or X represents a calcium or magnesium atom and n is an integer equal to 1, or
- hydrogen carbonates of formula (IVb): Y(HCO₃)m (IVb), in which Y represents a sodium or potassium atom and m is an integer equal to 1, or Y represents a calcium or magnesium atom and m is an integer equal to 2,
the neutralization being carried out so as to obtain a reaction medium, a 5% by weight dispersion of said reaction medium in water having a pH value comprised between 5.5 and 7.5,
c) A step c) of eliminating the sugar of formula (III), which has not reacted in step a), from the neutralized reaction medium, and
d) A step d) of recovering at least one composition (C) having a color equal to or less than 1.5 vcs.

2. The process according to claim 1, **characterized in that** said composition (C1) consists of a mixture of compounds represented by formulas (II1), (II2), (II3), (II4) and (II5):
R-O-(G)1-H (II1),
R-O-(G)2-H (II2),
R-O-(G)3-H (113),
R-O-(G)4-H (II4),
R-O-(G)5-H (II5),
in the respective molar proportions a1, a2, a3, a4 and a5, such that:
- the sum: a1+a2 + a3 + a4 + a5 is equal to 1, and
- the sum a1 + 2a2 + 3a3 + 4a4 + 5a5 is equal to x.

3. The process according to one of claims 1 or 2, **characterized in that** composition (C) comprises a quantity less than or equal to 2% by weight of the reducing sugar of formula (III):
H-O-(G)-H (III);
provided that the sum of the weight proportions of the compounds of formulas (I), (II) and (III) in composition (C) is equal to 100% by weight.

4. The process according to one of claims 1 to 3, **characterized in that** the basic agent (Ab) present in the aqueous solution is sodium carbonate of formula (IVa) in which X is the sodium atom and n is equal to 2.

5. The process according to one of claims 1 to 4, **characterized in that** it comprises, after step c), a complementary step of adjusting the pH value of a 5% by weight dispersion of the reaction medium in water by adding an aqueous solution comprising the basic agent (Ab) as defined previously so as to obtain a pH value comprised between 5.5 and 7.5.

6. The process according to one of claims 1 to 5, **characterized in that** the reducing sugar of formula (III) chosen for the glycosylation of step a) is chosen from the elements of the group consisting of glucose, xylose, arabinose and rhamnose.

7. The process according to one of claims 1 to 6, **characterized in that** step c) of eliminating the reducing sugar of formula (III) is carried out by filtration, centrifugation or decantation.

8. The process according to one of claims 1 to 7, **characterized in that** in step b) the aqueous solution of basic agent (Ab) comprises between 10% and 40% by weight of said basic agent (Ab).

9. The process according to one of claims 1 to 8, **characterized in that** step a) comprises the following successive sub-steps:
i) Introduction of an alcohol of formula (I) or a mixture of alcohols of formula (I), into a reactor (Ré) equipped with mechanical stirring and a vacuum device;
ii) Heating the alcohol of formula (I) to a temperature comprised between 80°C and 90°C under mechanical stirring;
iii) Loading the reducing sugar of formula (III) into the reactor (Ré);
iv) Introduction of at least one acid catalyst into the reactor (Ré),
v) Heating the reaction medium resulting from sub-step iv) and present in the reactor (Ré) under partial vacuum to a temperature comprised between 100°C and 110°C for the duration of the reaction, and
vi) Cooling the medium resulting from sub-step v) to a temperature comprised between 70°C and 80°C.

10. The process according to claim 9, **characterized in that** in formula (I) and/or in formula (II), the radical R is chosen from the following radicals: lauryl (or n-dodecyl) myristyl (or n-tetradecyl), n-pentadecyl, cetyl (or n-hexadecyl), n-heptadecyl, stearyl (or n-octadecyl), palmitoleyl (or 9-hexadecenyl), oleyl (or 9-octadecenyl), linoleyl (9,12-octadecadienyl), linolenyl (or 6,9,12-octadecatrienyl) n-nonadecyl, arachidyl (or n-eicosyl), behenyl (or n-docosyl), erucyl (13-docosenyl), or 12-hydroxystearyl.

11. The process according to claim 9, **characterized in that** in formula (I) and/or in formula (II), the radical R is chosen from the following radicals: 2-hexyl octyl, 2-hexyl decyl, 2-hexyl dodecyl, 2-octyl decyl, 2-octyl dodecyl, 2-decyl tetradecyl, isostearyl (or 16-methyl heptadecyl) or isomyristyl (or 13-methyl tridecyl).

12. The process according to one of claims 9 to 11, **characterized in that** during sub-steps ii) to iv) the reactor (Ré) is inerted under nitrogen.

13. The process according to one of claims 9 to 12, **characterized in that** it comprises between sub-steps ii) and iii) a sub-step of applying vacuum, preferably at a pressure less than or equal to 50 millibars.

14. The process according to one of claims 9 to 13, **characterized in that** step vi) is performed at atmospheric pressure.
